# EUROPEAN PATENT APPLICATION

(11) **EP 2 189 536 A1**
(43) Date of publication of application: **26.05.2010**
(21) Application number: 08169548.8
(22) Date of filing: 20.11.2008
(51) Int. Cl.: C12Q 1/04, C07K 7/06, C07K 5/06, C12Q 1/37, C07K 5/08, C07K 5/10

(54) **Rapid FRET based anthrax diagnosis**

(71) Applicant: Nederlandse Organisatie voor toegepast-natuurwetenschappelijk onderzoek TNO, 2628 VK Delft (NL)
(72) Inventor: Bikker, Floris Jacob, 2707 HW Gouda (NL); Kaman-van Zanten, Wendy Esmeralda, 2694 BK 's-Gravenzande (NL)
(74) Representative: Hatzmann, Martin

(57) **Abstract**

The invention comprises a substrate for detection of micro-organisms, wherein said substrate comprises a set of molecular markers linked to a di, tri- or tetrapeptide consisting of amino acids X1 and X2, X1, X2 and X3 or X1, X2, X3 and X4, respectively, in which one of them, for example X1 is a D-amino acid and the others, for example X2, X3 and X4 may be any D- or L-amino acid. Said substrate preferably is used for the detection of *Bacillus anthracis* and preferably a set of molecular markers linked to a dipeptide or tripeptide consisting of two amino acids X1 and X2 or three amino acids X1, X2 and X3. respectively, in which either
- one of X1, X2 and X3 is DLeu and one of the others is any L- or D-amino acid, preferably Leu, or
- one of X1, X2 and X3 is a D-amino acid and one of the others is preferably Leu.
The invention further comprises methods for detection of micro-organisms, specifically *Bacillus anthracis*, with the substrates of the invention.
The preferred substrates are, for example,
Ac-NH-Cys(S-Ac)-Leu-(D)Leu-Cys(S-Ac)-OH
and
FITC-Ahx-(D)Leu-Leu-Lys-DABCYL.

## Description

### Field of the invention

The present invention is in the field of diagnostics, more specifically detection of hazardous compounds or organisms, more specifically detection of micro-organisms, more specifically anthrax (*Bacillus anthracis*).

### Background

Anthrax is a zoonotic disease caused by the spore-forming bacterium *Bacillus anthracis. B. anthracis* spores remain viable in the environment for years, representing a potential source of infection. Anthrax occurs in humans in three clinical forms: inhalational, gastrointestinal, and cutaneous. Inhalational anthrax results from aerosolization of *B. anthracis* spores through industrial processing or intentional release. Gastrointestinal or oropharyngeal forms of the disease result from ingestion of infected undercooked or raw meat. Cutaneous anthrax is the most common type of naturally acquired anthrax infection and usually occurs after skin contact with contaminated products from infected animals. Historically, the case-fatality rate for cutaneous anthrax has been <1% with antibiotic treatment and 20% without antibiotic treatment (Brachman P.S. and Kaufmann A.F. In: Evans A.S., Brachman P.S., eds. Bacterial infections of humans. New York: Plenum Medical Book Company, 1998:95-111; Dixon T.C. et al, N. Engl. J. Med. 1999;341:815--26). Case-fatality rates for inhalational anthrax are high, even with appropriate antibiotics and supportive care (Jernigan J.A., et al. Emerg. Infect. Dis. 2001;7:933--44). Among the 18 cases of inhalational anthrax identified in the United States during the 20^{th} century, the overall case-fatality rate was >75%. After the biologic terrorism attack in fall 2001 in which *B. anthracis* spores were released through the mail, the case-fatality rate for patients with inhalational anthrax was 45% (5 of 11 cases) (Jernigan D.B., et al., Emerg. Infect. Dis. 2002;8:1019--28). The incubation period for anthrax is usually <2 weeks; however, because of spore dormancy and slow clearance from the lungs, the incubation period for inhalational anthrax can be prolonged for months. This phenomenon of delayed onset has not been recognized for cutaneous or gastrointestinal exposures. Discharges from cutaneous lesions are potentially infectious, but person-to-person transmission has been reported rarely. Person-to-person transmission of inhalational anthrax has not been documented.

*B. anthracis* is one of the biologic agents most likely to be used as a weapon because 1) its spores are highly stable; 2) the spores can infect through the respiratory route; and 3) the resulting inhalational disease has a high case-fatality rate. In 1979 an unintentional release of *B. anthracis* spores from a military microbiology facility in the former Soviet Union resulted in 69 deaths (Meselson M., et al. Science 1994;266:1202--8). The anthrax outbreak after *B*. *anthracis* spores were distributed through the U.S. mail system in 2001 further underscores the dangers of this organism as a terrorist threat.

After a terrorist attack, exposures to *B*. *anthracis* spores can occur through primary and secondary aerosols. Primary aerosols are dispersions of particles in air resulting from a biologic agent's initial release, whether through a disseminating device or through handling of an agent-containing package (e.g., in mechanical processing of mail). Secondary aerosols result from disruption and resuspension of settled particles.

Many detection systems for the presence of anthrax have been proposed. Archaic technologies such as staining have nowadays been replaced by more reliable molecular diagnostics, such as PCR (e.g. Makino, S.T. et al., J. Clin. Microbiol. 31:547-51, 1993); US 6,884,588; Qiagen's Real-Art™ B. anthracis PCR) and immunoassays (e.g. Swiecki, M.K., et al. J. Immunol. 176:6076-84, 2006; US 6,828,110; Response Medical Corp.'s RAMP™ Anthrax Assay). However, many of these systems can not differentiate between virulent and avirulent strains of *Bacillus anthracis.* The virulence of anthrax is mainly given by the presence of the pXO1 plasmid, on which plasmid the gene for the toxic proteins of anthrax are situated, of which one is denominated the lethal factor (LF-protein).

A relatively novel, very robust and highly reliable technology for the visualization of biological material is the FRET (Fluorescent Resonance Energy Transfer) technology. In this process, a photon from an energetically excited fluorophore, the 'donor', raises the energy state of an electron in another molecule, the 'acceptor', to higher vibrational levels of the excited state. As a result, the energy level of the donor fluorophore returns to the ground state, without emitting fluorescence. The acceptor thus functions as a quencher of the fluorescence. This mechanism is limited by the distance between the donor and the acceptor molecule. Typical effective distances between the donor and acceptor molecules are in the 10 to 100 Å range, which makes them useful in molecular diagnostics of e.g. nucleic acids and proteins.

The FRET technology has been used to detect *Bacillus anthracis* through coupling of FRET components to nucleic acids, especially those resulting from PCR products (Qi Y. et al., Appl. Environm. Microbiol. 67:3720-7, 2001; Patra G., et al., Annal. New York Acad. Sci. 969:106-11, 2002; Mathur, N. et al., J. Sensors 2008, Art. ID #270475). However, using FRET in combination with PCR still requires a high skill level of the technician performing the assay. Recently, FRET technology has been applied for the detection of anthrax through the proteolytic characteristics of the lethal factor protein (LF). In this assay a labeled substrate was added to the sample which is cleavable by LF (Cummings, R.T. et al., Proc. Natl. Acad. Sci. USA 99:6603-6, 2002). However, this assay gives rise to false positive signals since the cleavage characteristics for the substrate are not specific. Thus there is still need for an improved detection system for anthrax.

### SUMMARY OF INVENTION

### Technical Problem

The problem to be solved by the present invention is to provide the specific detection and diagnosis of anthrax *in situ*.

### Solution to Problem

The invention comprises substrates for detection, diagnosis, or identification of micro-organisms, or their enzymes, wherein said substrate comprises a set of molecular markers linked to a peptide consisting of at least one D-amino acid.

More specifically, the invention comprises a substrate for detection of micro-organisms, wherein said substrate comprises a set of molecular markers linked to a di-, tri- or tetrapeptide consisting of amino acids X1 and X2, X1, X2 and X3 or X1, X2, X3 and X4, respectively, in which one of, for example X1, is a D-amino acid and the others, for example, X2, X3 and X4 may be any D- or L-amino acid. Said substrate preferably is used for the detection of *Bacillus anthracis* and preferably a set of molecular markers linked to a dipeptide or tripeptide consisting of two amino acids X1 and X2 or three amino acids X1, X2 and X3, respectively, in which either
- one of X1, X2 and X3 is DLeu and one of the others is any L- or D-amino acid, preferably Leu, or
- one of X1, X2 and X3 is a D-amino acid and one of the others is preferably Leu.

In a preferred embodiment the invention comprises a substrate for fluorescent detection of *Bacillus anthracis* as defined above, wherein said set of molecular markers comprises a fluorescent label, preferably FITC (fluorescein-5-isothiocyanate), and a quencher for said fluorescent label, preferably DABCYL (4-((-4-(dimethylamino)-phenyl)-azo)-benzoic acid). More specifically, said substrate is: FITC - Ahx - X1- X2 - Lys - DABCYL wherein X1 and X2 are as defined in claim 1, wherein wherein Lys stands for Lysine, Val stands for valine and Gly stands for glycine, and Ahx stands for amino hexonic acid. In its most specific from in this embodiment, the substrate is chosen from the group consisting of :

| |
|---|
| FITC-Ahx-Leu-DLeu-Lys-DABCYL, |
| FITC-Ahx-DLeu-Leu-Lys-DABCYL, |
| FITC-Ahx-DLeu-DLeu-Lys-DABCYL, |
| FITC-Ahx-Leu-DLeu-Leu-Lys-DABCYL, |
| FITC-Ahx-Leu-DVal-Lys-DABCYL, |
| FITC-Ahx-Gly-DLeu-Lys-DABCYL, and |
| FITC-Ahx-Gly-DAla-Lys-DABCYL |

In an alternative embodiment, the invention comprises a substrate, wherein said substrate is: AcNH-Cys(S-Ac) - X1 - X2 - Cys(S-Ac)-OH, wherein X1 and X2 are as defined as above and wherein Cys(S-Ac) stands for thiol acetylated cysteine and AcNH for an acetylated amino terminus. More specifically in this embodiment the substrate is chosen from the group consisting of:

| |
|---|
| AcNH-Cys(S-Ac) -Leu-DLeu- Cys(S-Ac)-OH, |
| AcNH-Cys(S-Ac) -DLeu-Leu- Cys(S-Ac)-OH, |
| AcNH-Cys(S-Ac) -DLeu-DLeu- Cys(S-Ac)-OH, |
| AcNH-Cys(S-Ac) -Leu-DLeu-Leu- Cys(S-Ac)-OH, |
| AcNH-Cys(S-Ac) -Leu-DVal- Cys(S-Ac)-OH, |
| AcNH-Cys(S-Ac) -Gly-DLeu- Cys(S-Ac)-OH, and |
| AcNH-Cys(S-Ac) -Gly-DAla- Cys(S-Ac)-OH |

The invention further comprises a method for the detection of micro-organisms, in particular *Bacillus anthracis* in a sample comprising adding a substrate according to the invention to the sample and detecting fluorescence. In said method, the sample is preferably chosen from a body fluid, powder, water, food, medium or any other biological matrix..

The invention further specifically comprises a method for the identification *of Bacillus anthracis*^{*PXO1*+} in a sample comprising adding a substrate according to the invention to the sample and detecting fluorescence or a specific mass spectrum, or achieve detection by capillary electrophoresis .

Further part of the invention is the use of the substrate according to the invention for the detection and diagnosis *of Bacillus anthracis.*

### Advantageous Effects of Invention

First of all the invention offers a quick and efficient way of specifically detecting micro-organisms and specifically anthrax in a sample. Moreover, it is discriminative for anthrax that carries the PXO1 plasmid, i.e. anthrax that is virulent. Further, the detection method can be used in any sample with anthrax, without the need of (pre)-isolation.

### BRIEF DESCRIPTION OF DRAWINGS

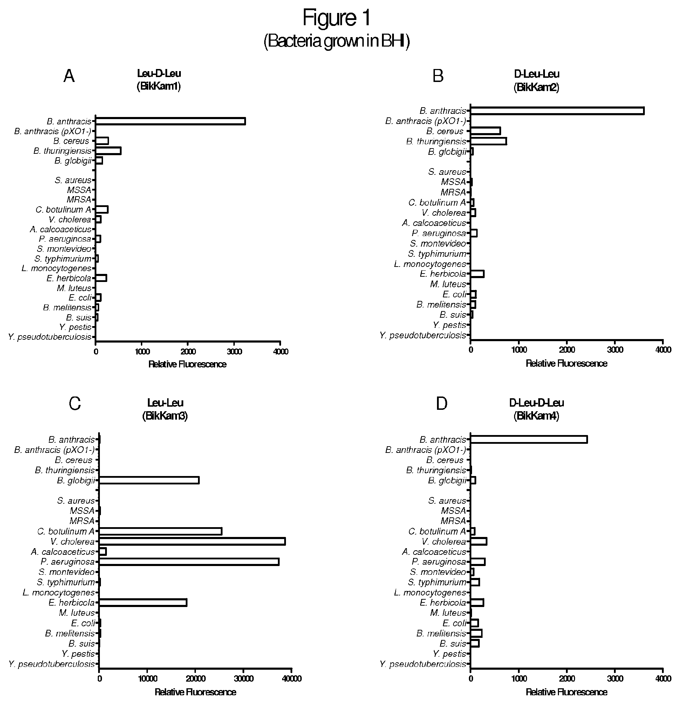

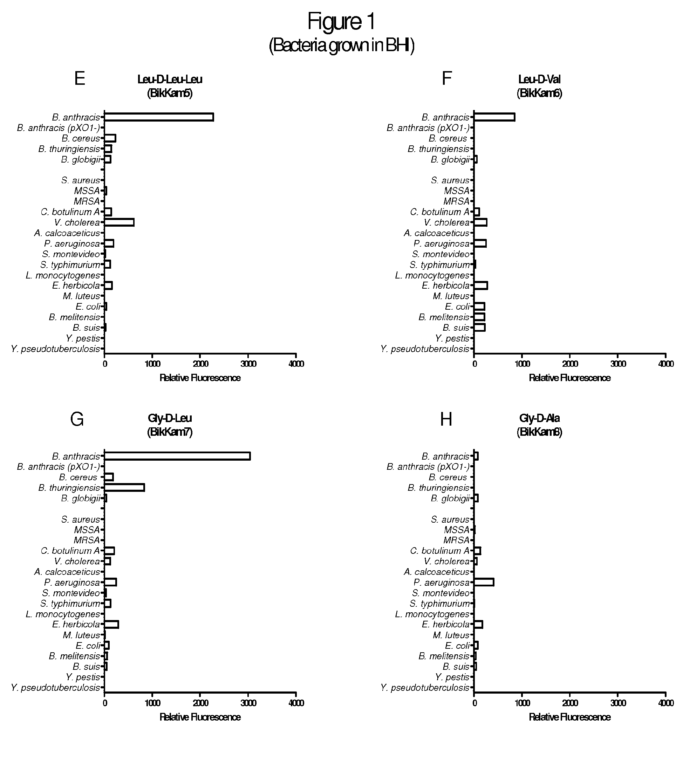

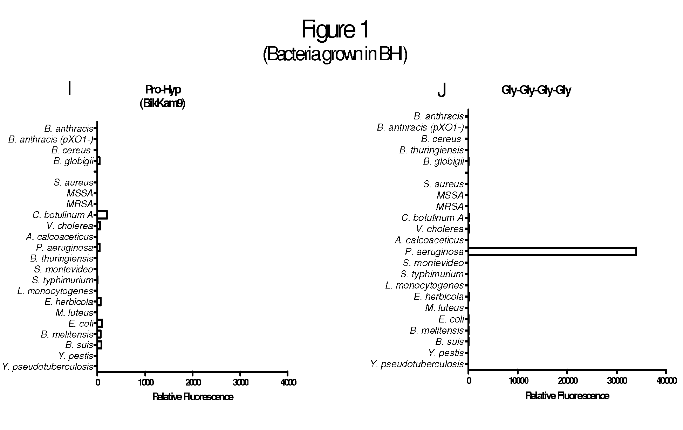

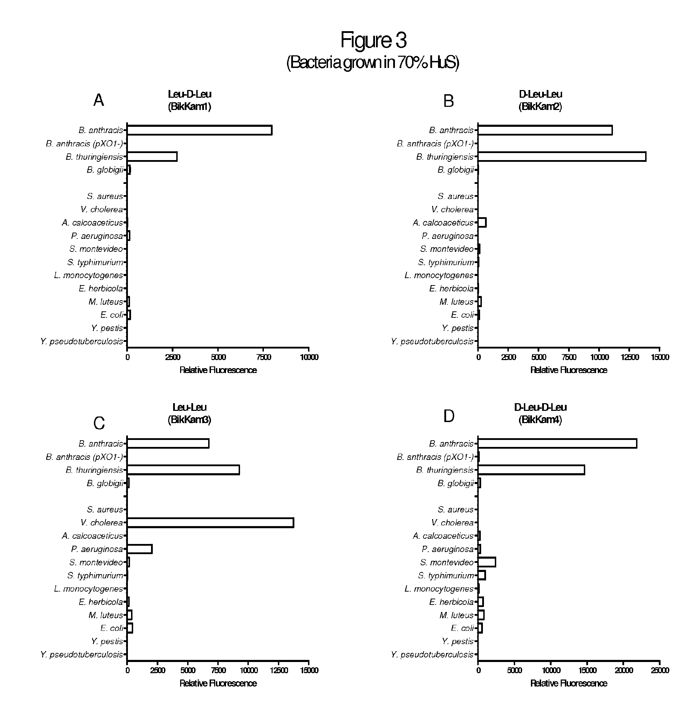

**Fig. 1****. Fluorescent signal of FRET after 60 minutes of invented substrates by enzymes of bacteria grown in bacterial growth medium (BHI) (*in vitro*)**
A: BikKam1 is specifically cleaved by a (virulent) *B. anthracis-* derived enzyme.
B: BikKam2 is specifically cleaved by a (virulent) *B. anthracis-* derived enzyme. A weak signal was observed for *B. cereus* and *B. thuringiensis.*
C: *B. anthracis*-derived enzymes do not digest BikKam3. Otherwise enzymes derived from *B. globigii, C. botulinum, P. aeruginosa, V. cholerae* and *E. herbicola* show activity on the substrate, indicating that the enzymes, more specifically, this approach, enables broad-spectrum detection of micro-organisms (see also Fig 3C).
D: BikKam4 is specifically cleaved by a (virulent) *B. anthracis-* derived enzyme.
E: BikKam5 is specifically cleaved by a (virulent) *B. anthracis-* derived enzyme. Some cleavage is observed for *V. cholerae* as well.
F: BikKam6 is relatively moderate, but specifically cleaved by a (virulent) *B. anthracis-* derived enzyme.
G: BikKam7 is specifically cleaved by a (virulent) *B. anthracis-* derived enzyme. A weak signal was observed for *B. thuringiensis.*
H. No significant enzyme activity could be measured using BikKam8 with the currently used micro-organisms.
I. No significant enzyme activity could be measured using BikKam9 with the currently used micro-organisms.
J: FITC-Ahx-Gly-Gly-Gly-Gly -Lys-DABCYL is specifically cleaved by a *P. aeruginosa-* derived enzyme.

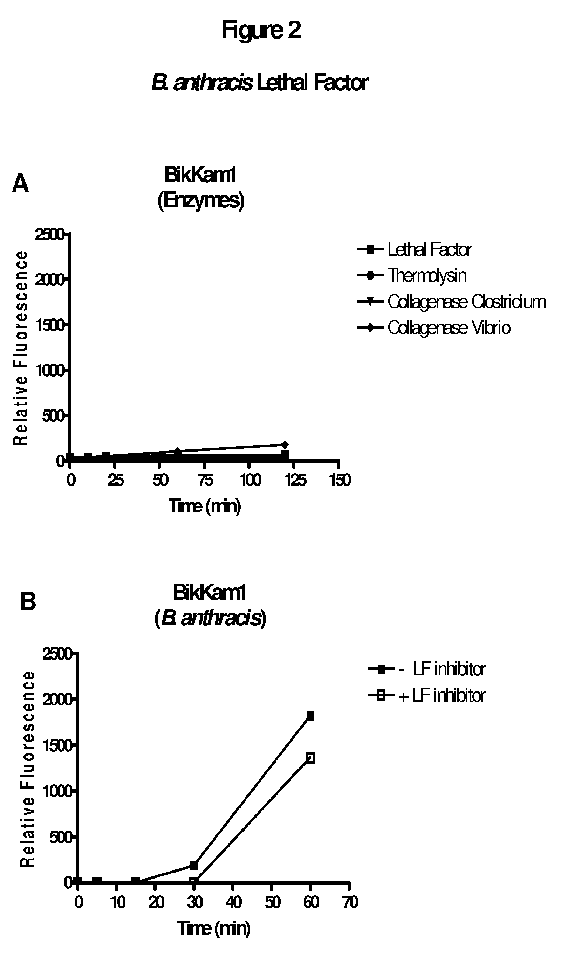

**Fig. 2****. The enzyme that cleaves BikKam1 is situated on the PXO1 plasmid, but is not Lethal Factor.**
A: Substrate BikKam1 was added to several purified enzymes: Lethal Factor, thermolysin, collegase (from *Clostridium sp*.) and collegenase (from *Vibrio sp*). No signal with all enzymes was observed, underlining the specificity of BikKam1 for a *B. anthracis-derived* enzyme, but which is not LF.
B: BikKam1 was added to LF. Supplemented LF inhibitor did not result in a significant decrease of substrate cleavage, underlining the specificity of BikKam1 for a *B. anthracis-derived* enzyme, but which is not LF.

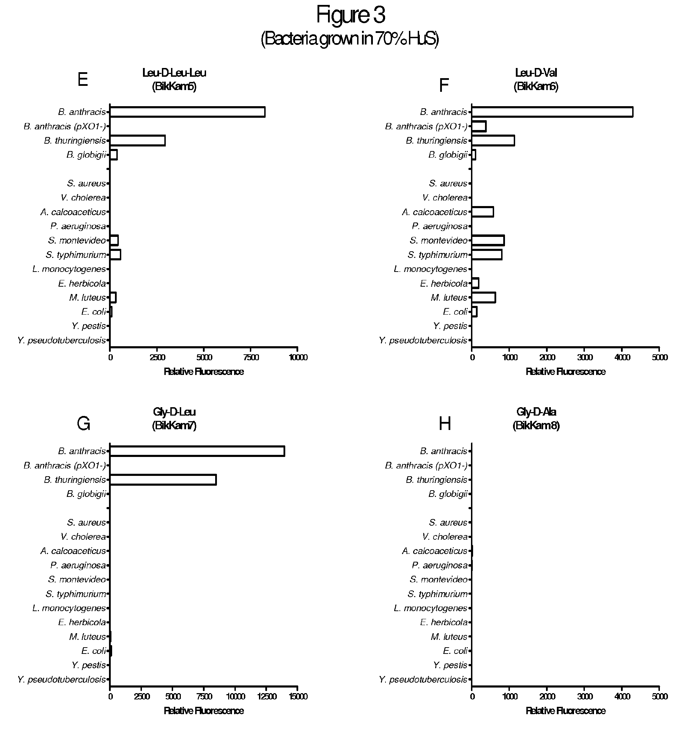

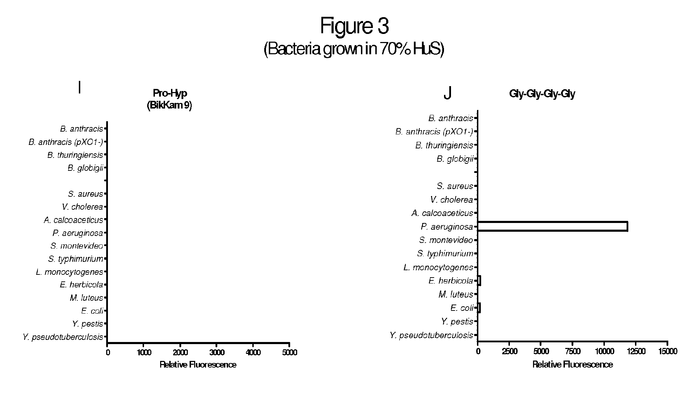

**Fig. 3****. Fluorescent signal of FRET after 60 minutes of incubation of substrates with enzymes of bacteria grown in human serum (*ex vivo*),**
A: BikKam1 is specifically cleaved by a (virulent) *B. anthracis-* derived enzyme. Some weak activity was observed for *B. thuringiensis* as well.
B: BikKam2 is specifically cleaved by a (virulent) *B. anthracis-* derived enzyme and a *B. thuringiensis*-derived enzyme.
C: BikKam3 is specifically cleaved by a (virulent) *B. anthracis-* derived enzyme, a *B. thuringiensis*-derived enzyme and a *V. cholerae-derived* enzyme. Some weak activity was observed for *P. aeruginosa.* These data provide evidence for differential use of the invented substrates /approach; identical substrates show differential activity when grown in either BHI or Human serum (compare with Fig 1 C).
D: BikKam4 is specifically cleaved by a (virulent) *B. anthracis-* derived enzyme and a *B. thuringiensis*-derived enzyme.
E: BikKam5 is specifically cleaved by a (virulent) *B. anthracis-* derived enzyme. Some weak activity was observed for *B. thuringiensis.*
F: BikKam6 is moderately specifically cleaved by a (virulent) *B. anthracis-*derived enzyme. Some weak background with other bacteria was observed at 60 min. Measurements at earlier time points *e.g.* 30 minutes hardly revealed any background with bacteria other than (virulent) *B. anthracis* (not shown). G: BikKam7 is specifically cleaved by a (virulent) *B. anthracis-* derived enzyme and to a lesser extend by a *B. thuringiensis*-derived enzyme.
H. No significant enzyme activity could be measured using BikKam8 with the currently used micro-organisms.
I. No significant enzyme activity could be measured using BikKam9 with the currently used micro-organisms.
J: FITC-Ahx-Gly-Gly-Gly-Gly -Lys-DABCYL is specifically cleaved by a *P. aeruginosa-* derived enzyme.

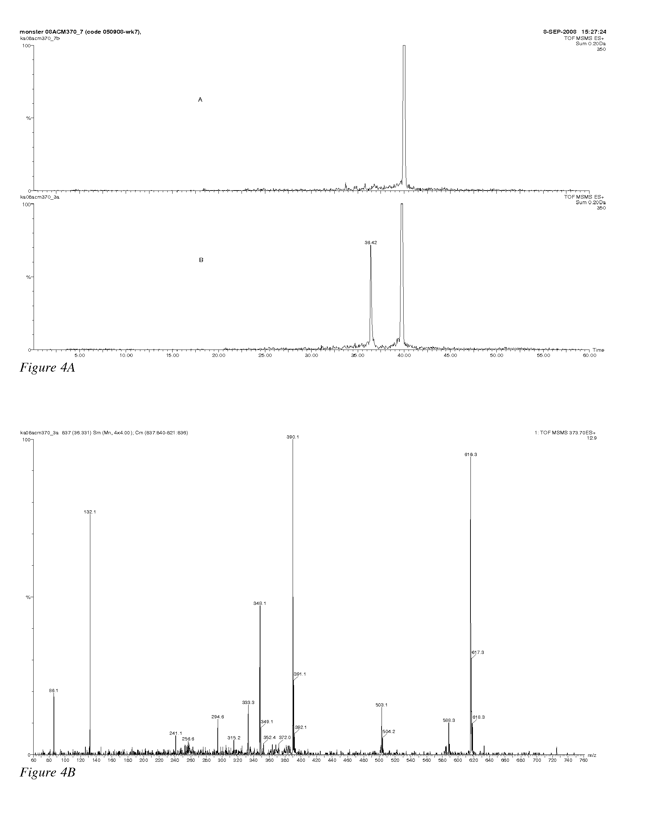

Fig. 4. A: Extracted ion chromatograms of ions m/z 132.1+348.1+390.1+503.2+616.2, fragments in the product ion mass spectrum of MH₂²⁺ 374.2 of [FITC]-Ahx-Leu-D-Leu. Upper panel: serum of uninfected mouse, lower panel: serum of infected mouse.
B: product ion mass spectra of MH₂²⁺ 374.2 of [FITC]-Ahx-Leu-D-Leu.

### DESCRIPTION OF EMBODIMENTS

The current invention is directed to a method for the detection or diagnosis of anthrax based on an interaction between a substrate and a proteolytic enzyme that is present in the bacterium *Bacillus anthracis.* In order to enable detection, the substrate comprises a set of molecular markers that are used to detect a difference between the intact substrate and the substrate that is cleaved by the specific action of the *B. anthracis* protease.

Thus, the invention comprises a substrate for detection of micro-organisms, wherein said substrate comprises a set of molecular markers linked to a di-, tri- or tetrapeptide consisting of amino acids X1 and X2, X1, X2 and X3 or X1, X2, X3 and X4, respectively, in which one of them , for example X1, is a D-amino acid and the others, for example, X2, X3 and X4 may be any D- or L-amino acid.

Central in the substrate is a sequence of two or three amino acids comprised of either one D-leucine in combination with another D- or L-amino acid(s), preferably L-leucine, in either direction (*i.e*. Leu-DLeu or DLeu-Leu), or a D-amino acid in combination with a leucine residue, to which the set of molecular markers is linked. It has appeared that these sequences are very specifically cleaved by a hitherto unknown enzyme of *B. anthracis.* As can be seen in the experimental part, the substrate is specific in that it is hardly cleaved by closely related bacteria (*B. thuringiensis* and *B. cereus*) and not at all by *B. globigii, B. suis, E. coli* and *S. typhimurium*.

Also, these sequences seem to be specific in the sense that a change therein causes loss of the specific characteristics. As can be seen in the experimental part, a sequence with Leu-Leu is not cleaved by *B. anthracis,* but is cleaved by some other bacteria (*V. cholerae, P. aeruginosa, E. herbicola, C. botulinum* and *B. globigii*).

The dipeptide that is preferred is the dipeptide Leu-DLeu (or the reverse (DLeu-Leu). It appears that this sequence is not only the best performing dipeptide for the detection of *B. anthracis,* but that it can also be used for the *ex vivo* diagnosis of anthrax.

The molecular markers linked to the dipeptide of the invention may be any set of markers which can discriminate between the intact molecule and the cleaved molecule. A simple detection method may be e.g. mass spectrography in which the mass of the fragments is easily discriminated from the mass of the intact molecule. In such an assay, it is also unnecessary to have two molecular markers, since the presence of a marker at one side of the molecule will be sufficient for the detection.

Preferred is an assay in which the presence of the cleaved peptide will be directly visible, such as by fluorescence. Especially advantageous is an assay that is based on the FRET technology. In such an assay a fluorophore is attached to one side of the molecule to be cleaved and a quencher for said fluorophore is attached to the other side of the molecule, but at a distance that the quencher is still able to quench the fluorophore in the intact molecule. Normally in FRET systems the distance between the fluorophore and the quencher may not exceed 100 Å. The choice of the fluorophore-quencher pair may be made from several available pairs, see e.g. Marras, S.A.E. (Meth. Mol. Biol. 335:3-16, 2006), where in table 2, 3 and 4 several fluorophores and their quenchers have been mentioned. These fluorophores and quenchers can all be used in the present invention. Preferably, FITC (fluorescein-5-isothiocyanate) is used as fluorophore and DABCYL (Dbc, 4-((-4-()dimethylamino)-phenyl)-azo)-benzoic acid) is used as the corresponding quencher.

The substrate flanked by the fluorophore-quencher pair would then be depicted as:
Marker1 - X1 - X2 - Marker2 or Marker1 - X1 - X2 - X3-Marker2
in which either
- one of X1, X2 or X3 is DLeu and one of the other(s) is be any L- or D-amino acid, preferably an aliphatic amino acid, more preferably Leu, or
- one of X1, X2 and X3 is a D-amino acid, preferably a D-aliphatic amino acid, and preferably one of the other(s) is Leu, and
   Marker1 and Marker2 form the fluorophore-quencher pair.
   In case the fluorophore-quencher pair is FITC and DABCYL, the formula will be represented by:
   FITC - Ahx - X1 - X2 - Lys - DABCYL,
   in which X1 and X2 are as defined above, Ahx is aminohexonic acid, Lys is the amino acid lysine.
   In the FRET assay, the substrate according to the invention is added to the sample and fluorescence will indicate that the fluorophore is no longer quenched by the quencher, which means that the substrate has been cleaved, which means that *B. anthracis* is present in the sample. Thus, this is a simple assay, with direct visual read-out of the presence or absence of anthrax in the sample.
   Alternatively, visible detection of protease activity can be performed through a novel assay based on color changes of gold colloids by cysteinyl derivatives (see: Guarise, C. et al., Proc. Natl. Acad. Sci. 103:3978-82, 2006). In this assay, the ability of a peptide having two terminal acetylated cysteines to interfere with the aggregation of nanometer-sized gold colloids and thereby introducing a shift in visible color, versus the lack of ability to introduce a color change of a peptide with only one such an acetylated cysteine terminal is used. The substrate of the present invention for such an assay would then be:
   AcNH-Cys(S-Ac) - X1 - X2 - Cys(S-Ac)-OH or AcNH-Cys(S-Ac) - X1 - X2 - X3 - Cys(S-Ac)-OH
   in which either
- one of X1, X2 and is DLeu and one of the other(s) is any L- or D-amino acid, preferably an aliphatic amino acid, more preferably Leu, or
   - one of X1, X2 and X3 is a D-amino acid, preferably a D-aliphatic amino acid, and preferably one of the other(s) is Leu,
   - in which S-Ac is thiol-acetylated cysteine and AcNH is the acetylated amino terminal end.
   - In this assay, the substrate is added to the sample and then the total of sample and substrate is added to a suspension of gold nanoparticles. If the color of the gold nanoparticles changes, then intact substrate is still available, meaning that the substrate has not been cleaved, which indicates that no *B. anthracis* has been present in the sample. No color change indicates a cleaved substrate and presence of anthrax.

The detection method of the invention is suitable for the detection of *B*. *anthracis,* but also other micro-organisms can be detected, as is shown in the examples, in several kinds of samples. Most preferable, a sample from a patient suspected of anthrax is used. Such a sample can be a blood, serum or plasma sample or can be derived from other body fluids, such as urine, sputum, lymph fluid, etc.

Alternatively, the sample can be taken from an environmental source, such as soil, water or air. In the latter case, preferably a filter is used through which the air is ventilated and in which the micro-organisms are collected. It is also possible, of course, to take a sample from any suspected powder (such as a powder in an envelope in the mail), from food that may be contaminated with anthrax or from medium in which micro-organisms are normally grown. After the sample is taken, it may be directly reacted with a substrate of the invention and fluorescence or other visual signal may be detected in *B*. *anthracis* is present. In this way a rapid diagnosis of the presence of anthrax may be obtained.

It has further appeared that the enzymatic activity from *B. anthracis* that is responsible for cleavage of the dipeptide is resided on the PXO1 plasmid. Since this plasmid also harbours the toxicity genes of anthrax, such as the lethal factor protein, the present method of detection is also specific for those strains *of B. anthracis* that are harmful. Hence, it is also possible with the present detection method to indentify *B. anthracis*^{*PXO1*+} strains.

### EXAMPLES

### Materials and Methods

### Bacteria

Bacteria were grown overnight in Brain Heart Infusion (BHI) medium or 70% Human serum (HuS) at the appropriate growth temperature. Next day the bacteria were pelleted by centrifugation for 10 min at 10 000 rpm.

Supernatant was sterilized by filtration through an 0.22 uM filter (MilliPore). For mass spectrometric analysis and confirmation of the *in vitro* and *ex vivo* results sera of mice that were suffering a *B. anthrax* infection was filtrated on a Waters Sep-Pak classic cartridge WAT051910 C18 column.

### Reagents

FRET peptides were synthesized by PepScan (The Netherlands). Identity and purity were verified by mass spectrometry and reverse phase HPLC. Anthrax Lethal Factor (LF) was purchased from List Laboratories. Thermolysin and Collagenase *Clostridium* were purchased from Sigma. Collagenase *Vibrio*/ Dispase was purchase from Roche. LF inhibitor 12155 was synthesized by Pyxis (The Netherlands). Nine experimental peptides were synthesized and reacted with FITC and DABCYL to form the following reagents:

| Internal code | Formula |
|---|---|
| | |
| BikKam 1 | FITC-Ahx-Leu-DLeu-Lys-DABCYL |
| BikKam 2 | FITC-Ahx-DLeu-Leu-Lys-DABCYL |
| BikKam 3 | FITC-Ahx-Leu-Leu-Lys-DABCYL |
| BikKam 4 | FITC-Ahx-DLeu-DLeu-Lys-DABCYL |
| BikKam 5 | FITC-Ahx-Leu-DLeu-Leu-Lys-DABCYL |
| BikKam 6 | FITC-Ahx-Leu-DVal-Lys-DABCYL |
| BikKam 7 | FITC-Ahx-Gly-DLeu-Lys-DABCYL |
| BikKam 8 | FITC-Ahx-Gly-DAla-Lys-DABCYL |
| BikKam 9 | FITC-Ahx-Pro-Hyp-Lys-DABCYL |

| | |
|---|---|
| Ahx = aminohexonic acid, Leu = L-leucine, DLeu = D-leucine, Lys = L-lysine, DVal = D-valine, Gly = L-glycine, DAla = D-alanine, Pro = L-proline, Hyp = hydroxyproline. | |

### FRET assay

Assay reactions were preformed in blackwell, clear bottom 96-well plates (Corning). For the enzyme assay 10 uM of each of the above mentioned reagents was incubated at 37 °C in presence of Thermolysin (1 ug), Collagenase *Clostridium* (10 ug), Collagenase *Vibrio*/ Dispase (5 ug) or LF (1 ug). Enzyme activity in bacterial supernatants was determined by incubating 16 uM FRET peptide with 100 uL filtered supernatant at 37 °C. Filtered BHI medium or 70% HuS was used as control. Plates were read with 10 min intervals on a Cytofluor 4000 (Applied Biosystems) with excitation using a 485 nm filter and emission using a 530 nm filter. The results are shown in Figure 1, 2 and 3.

### Mass Spectrometry

SepPak fractions (Waters Sep-pak classic cartridge C18) of BikKam1 1): in BHI with and without *B. antracis;* and 2): incubated with serum of an infected and control mice were analyzed for the presenceof [FITC]-Ahx-Leu-D-Leu and [dabcyl] -K(NH₂).

These samples were analysed with ES-LC-MS/MS under the following conditions:
• Elution liquid A: H₂O (0.2 % formic acid) Elution liquid B: CH₃CN (0.2 % formic acid)
•

| Gradient: | time (min.) | % A | % B | flow (ml/min.) |
|---|---|---|---|---|
| | 0 | 100 | 0 | 0.6 |
| | 45 | 10 | 90 | 0.6 |

The flow of 0.6 ml/min. was reduced by an LC-packings splitter to about 40 µl/min.
• Column: PepMap C₁₈; 3 µm; 15 cm x 1 mm Size: 10 en 50 µl

Spectra (MS/MS) of the produced ions were recorded from the double charged molecular ion (MH₂²⁺ 199.1) of [dabcyl]-K(NH₂) with a cone voltage of 20 V and a collision energy of 10 - 11 eV. Spectra were recorded from the double charged molecular ion (MH₂²⁺ 374.2) of [FITC]-Ahx-Leu-D-Leu with a cone voltage of 20-25 V and a collision energy of 13 eV.

The argon gas pressure was 10⁻⁴ mBar.

The results in Fig. 4 show that compared to uninfected mice (Fig 4A) in *B*. *anthracis* infected mice an additional peak is observed, representing in this example [FITC]-Ahx-Leu-D-Leu

## Claims

1. Substrate for detection of micro-organisms, wherein said substrate comprises a set of molecular markers linked to a di-, tri- or tetrapeptide consisting of amino acids X1 and X2, X1, X2 and X3 or X1, X2, X3 and X4, respectively, in which one of them, for example X1 is a D-amino acid and the others, for example X2, X3 and X4 may be any D- or L-amino acid

2. Substrate for detection of micro-organisms, in particular *Bacillus anthracis,* according to claim 1, wherein said substrate comprises a set of molecular markers linked to a dipeptide or tripeptide consisting of two amino acids X1 and X2 or three amino acids X1, X2 and X3, respectively, in which either
- one of X1, X2 and X3 is DLeu and one of the others is any L- or D-amino acid, preferably Leu, or
- one of X1, X2 and X3 is a D-amino acid and one of the others is preferably Leu.

3. Substrate for fluorescent detection of *Bacillus anthracis* according to claim 1 or 2, wherein said set of molecular markers comprises a fluorescent label, preferably FITC (fluorescein-5-isothiocyanate), and a quencher for said fluorescent label, preferably DABCYL (4-((-4-(dimethylamino)-phenyl)-azo)-benzoic acid).

4. Substrate according to any of claims 1-3, wherein said substrate is:
FITC - Ahx - X1- X2 - Lys - DABCYL wherein X1 and X2 are as defined in claim 1, wherein wherein Lys stands for Lysine, Val stands for valine and Gly stands for glycine, and Ahx stands for amino hexonic acid.

5. Substrate according to any of claims 1-4, wherein said substrate is chosen from the group consisting of :
| |
|---|
| FITC-Ahx-Leu-DLeu-Lys-DABCYL, |
| FITC-Ahx-DLeu-Leu-Lys-DABCYL, |
| FITC-Ahx-DLeu-DLeu-Lys-DABCYL, |
| FITC-Ahx-Leu-DLeu-Leu-Lys-DABCYL, |
| FITC-Ahx-Leu-DVal-Lys-DABCYL, |
| FITC-Ahx-Gly-DLeu-Lys-DABCYL, and |
| FITC-Ahx-Gly-DAla-Lys-DABCYL |

6. Substrate according to claim 1 or 2, wherein said substrate is:
AcNH-Cys(S-Ac) - X1- X2 - Cys(S-Ac)-OH,
wherein X1 and X2 are as defined in claim 3 and wherein Cys(S-Ac) stands for thiol acetylated cysteine and AcNH for an acetylated amino terminus.

7. Substrate according to any of claims 1, 2 or 6, wherein said substrate is chosen from the group consisting of:
| |
|---|
| AcNH-Cys(S-Ac) -Leu-DLeu- Cys(S-Ac)-OH, |
| AcNH-Cys(S-Ac) -DLeu-Leu- Cys(S-Ac)-OH, |
| AcNH-Cys(S-Ac) -DLeu-DLeu- Cys(S-Ac)-OH, |
| AcNH-Cys(S-Ac) -Leu-DLeu-Leu- Cys(S-Ac)-OH, |
| AcNH-Cys(S-Ac) -Leu-DVal- Cys(S-Ac)-OH, |
| AcNH-Cys(S-Ac) -Gly-DLeu- Cys(S-Ac)-OH, and |
| AcNH-Cys(S-Ac) -Gly-DAla- Cys(S-Ac)-OH |

8. Method for the detection of micro-organisms, in particular *Bacillus anthracis* in a sample comprising adding a substrate according to any of claims 1-7 to the sample and detecting fluorescence.

9. Method according to claim 8, wherein the sample is chosen from a body fluid, powder, water, food, medium or any other biological matrix.

10. Method for the identification *of Bacillus anthracis*^{*PXO1*+} in a sample comprising adding a substrate according to any of claims 1-6 to the sample and detecting fluorescence or a specific mass spectrum, or achieve detection by capillary electrophoresis .

11. Use of the substrate according to any of claims 1-7 for the detection and diagnosis *of Bacillus anthracis.*
